# EUROPÄISCHE PATENTSCHRIFT

(11) **EP 3 164 707 B1**
(45) Veröffentlichungstag und Bekanntmachung des Hinweises auf die Patenterteilung: **03.04.2019**
(21) Anmeldenummer: 16713723.1
(22) Anmeldetag: 11.03.2016
(51) Int. Cl.: G01N 33/36, B65H 63/08, D01H 13/00, B65H 57/16

(54) **HALBAUTOMATISCHE GARNWECHSELVORRICHTUNG FÜR EIN GARNPRÜFGERÄT**
SEMIAUTOMATIC THREAD-CHANGING DEVICE FOR A THREAD-TESTING DEVICE
DISPOSITIF DE CHANGEMENT DE FIL SEMI-AUTOMATIQUE POUR UN APPAREIL DE TEST DE FIL

(30) Priorität: 20.03.2015 CH 4102015
(43) Veröffentlichungstag der Anmeldung: 10.05.2017
(73) Patentinhaber: Uster Technologies AG, 8610 Uster (CH)
(72) Erfinder: SCHEIBER, Patrik, 8155 Niederhasli (CH); KUSTER, Martin, 8733 Eschenbach (CH); DE VRIES, Loris, 8625 Gossau (CH); KOLLER, Beat, 8638 Goldingen (CH); FENNER, Jürg, 8600 Dübendorf (CH)
(74) Vertreter: Pliska, Pavel
(86) Internationale Anmeldenummer: PCT/CH2016/000045
(87) Internationale Veröffentlichungsnummer: WO 2016/149845

(56) Entgegenhaltungen:
- EP-A2- 0 585 555
- CH-A- 519 717
- DE-A1- 4 437 026
- Statex Engineering Ltd: "Statex Evenness Tester-600", , 12. März 2012 (2012-03-12), XP054975929, Gefunden im Internet: URL:https://www.youtube.com/watch?v=7MOA4p LY0vc [gefunden am 2015-06-18]

## Beschreibung

### FACHGEBIET

Die vorliegende Erfindung liegt auf dem Gebiet der textilen Qualitätsprüfung und bezieht sich auf eine halbautomatische Garnwechselvorrichtung für ein Garnprüfgerät, gemäss dem Oberbegriff des ersten Patentanspruchs. Ferner bezieht sich die Erfindung auf ein derartiges Garnprüfgerät gemäss dem Oberbegriff eines weiteren Patentanspruchs. Das erfindungsgemässe Garnprüfgerät ist als eigenständige, nicht in einen Produktionsprozess eingebundene Vorrichtung konzipiert und somit im Off-line-Betrieb, bspw. im Textillabor, einsetzbar.

### STAND DER TECHNIK

Gamprüfgeräte werden eingesetzt, um die Qualität von Garnen zu prüfen. Es geht darum, einerseits eine gleich bleibende Qualität der herzustellenden textilen Produkte zu erreichen, andererseits aber auch darum, den textilen Produktionsprozess anhand von Stichproben off-line zu überwachen.

Die bekannten Gamprüfgeräte dieser Art arbeiten nach dem Durchlaufprinzip, d. h. das zu prüfende Garn wird in einem einzigen Prüfdurchlauf von einer Garnspule abgezogen und durchläuft in serieller Weise im Garnprüfgerät eine Anordnung von Sensoren, um die zu bestimmenden Parameter zu messen. Dies ist grundsätzlich ein kontinuierlicher Prozess. Gamprüfgeräte neueren Typs können automatisch eine Mehrzahl von Garnproben nacheinander abarbeiten, die vorgängig von einer Bedienperson manuell eingelegt werden müssen. Der Probenwechsel, die Einführung der jeweiligen Garnprobe in einen Garnpfad und ihre Ausmessung erfolgen dann automatisch ohne Eingriff der Bedienperson.

Ein Garnprüfgerät mit einer halbautomatischen Garnwechselvorrichtung und einer automatischen Einführungsvorrichtung ist in der US-3,805,607 A offenbart. Die Garnwechselvorrichtung gemäss der US-3,805,607 A ist halbautomatisch, was bedeutet, dass eine Mehrzahl von zu prüfenden Garnproben zwar manuell in die Garnwechselvorrichtung eingelegt werden müssen, die Position der Garnwechselvorrichtung im Prüfbetrieb dann aber automatisch auf den Garnpfad und die Einführungsvorrichtung ausrichtbar ist. Die zu prüfenden Garne befinden sich auf Spulen, die auf einem sich in der Nähe des Prüfgerätes befindlichen Spulenwagen gelagert sind. Die Garnwechselvorrichtung bewahrt die Mehrzahl von Garnenden auf einem Schlitten auf, wo sie äquidistant und parallel zueinander eingespannt sind. Jede Garnprobe wird in zwei fluchtenden Garnklemmen eingeklemmt, die auf vertikal übereinander liegenden horizontalen Stangen angeordnet sind. Eine Garnklemme beinhaltet zwei Zylinder, die auf der betreffenden Stange in horizontaler Richtung verschiebbar und mit einer vorgespannten Druckfeder gegeneinander gedrückt werden, so dass die Garnprobe dazwischen eingeklemmt werden kann. Der Schlitten kann mittels eines Motors in horizontaler Richtung verschoben werden, wobei ein Ratschenmechanismus für eine Vorschubbewegung um jeweils eine Äquidistanz sorgt. Die sich jeweils in einer Prüfposition befindliche Garnprobe wird zwischen den beiden horizontalen Stangen von der Einführungsvorrichtung ergriffen und mittels einer Drehbewegung automatisch in einen Garnpfad des Prüfgerätes eingelegt, worauf die Garnprüfung beginnen kann.

Die US-2008/0209998 A1 zeigt ein anderes Garnprüfgerät mit einer halbautomatischen Garnwechselvorrichtung und einer automatischen Einführungsvorrichtung, nämlich das Garnprüfgerät USTER® *TESTER 5* der Anmelderin des vorliegenden Schutzrechtes. Der USTER® *TESTER 5* hat eine Garnwechselvorrichtung, in welcher jede Garnprobe nur in einer einzigen, an einer vertikalen Vorderfläche der Garnwechselvorrichtung angebrachten Garnklemme eingeklemmt wird. Jede Garnklemme beinhaltet zwei federbeaufschlagte Teller, zwischen denen die Garnprobe eingeklemmt wird. Die Klemmflächen der Teller sind parallel zur Vorderfläche gerichtet, so dass die Garnproben in einer Richtung parallel zur Vorderfläche in die Garnklemmen eingelegt werden. Die Einführungsvorrichtung vollführt keine Dreh-, sondern eine Linearbewegung.

Das Garnprüfgerät Statex Evenness Tester - 600 der Statex Engineering (P) Ltd., Comibatore (Indien), hat eine Garnwechselvorrichtung mit einer Mehrzahl von nebeneinander angeordneten Einspannstellen für die Garnproben. Jede Einspannstelle beinhaltet eine Garneinlauföse und zwei stromabwärts der Garneinlauföse angeordnete Garnklemmen. Die Garneinlauföse und die beiden Garnklemmen liegen im Wesentlichen in einer Frontebene des Garnprüfgerätes, und die eingespannten Garnproben verlaufen vertikal von oben nach unten. Die Klemmflächen der Garnklemmen sind, wie beim USTER® *TESTER 5,* parallel zur Frontebene gerichtet, so dass die Garnproben in einer Richtung parallel zur Frontebene in die Garnklemmen eingelegt werden.

Ein Nachteil der obigen Garnwechselvorrichtungen sind die Umständlichkeit und der grosse Zeitaufwand bei ihrer Beschickung. Die Bedienperson muss zuerst alle Garnproben von der Rückseite her lose in der richtigen Reihenfolge auf der Garnwechselvorrichtung bereitlegen. Danach geht sie um das Garnprüfgerät herum zu seiner Vorderseite und klemmt jedes der bereitliegenden Garnenden in die entsprechenden Garnklemmen ein. Zum Einklemmen muss jedes Garnende zuerst in die Nähe seiner Garnklemme gebracht, parallel zur Frontebene ausgerichtet und schliesslich parallel zur Frontebene in die Garnklemme hinein verschoben werden.

### DARSTELLUNG DER ERFINDUNG

Es ist eine Aufgabe der vorliegenden Erfindung, die halbautomatischen Garnwechselvorrichtungen, wie sie aus der US-3,805,607 A, aus dem Garnprüfgerät USTER® *TESTER 5* oder aus dem Garnprüfgerät Statex Evenness Tester - 600 bekannt sind, weiter zu verbessern. Insbesondere soll das manuelle Einlegen der Garnproben einfacher und zeitsparender sein. Dabei soll die Ergreifbarkeit durch eine automatische Einführungsvorrichtung mindestens so zuverlässig oder zuverlässiger sein wie bei den Garnwechselvorrichtungen gemäss dem Stand der Technik. Eine weitere Aufgabe ist es, ein Garnprüfgerät mit einer verbesserten Garnwechselvorrichtung zur Verfügung zu stellen.

Diese und andere Aufgaben werden durch die im ersten Patentanspruch definierte Garnwechselvorrichtung und das in einem weiteren Anspruch definierte Garnprüfgerät gelöst. Vorteilhafte Ausführungsformen sind in den abhängigen Patentansprüchen angegeben.

Der Erfindung liegt die Idee zugrunde, diejenigen Elemente, die zum Einspannen der Garnproben benötigt werden, auf einer Einspanngeraden anzuordnen und sie so auszugestalten, dass eine Garnprobe jeweils durch eine einzige, einfache Handbewegung einlegbar ist. Das Einlegen der Garnprobe erfolgt in einer Einlegerichtung senkrecht zu einer Einspannebene, die durch alle Einspanngeraden aufgespannt ist. Bei den genannten Elementen zum Einspannen handelt es sich um eine Garneinlauföse und zwei Garnklemmen.

Die erfindungsgemässe halbautomatische Garnwechselvorrichtung dient zur Bereitstellung einer Mehrzahl von Garnproben für die Prüfung in einem Garnprüfgerät. Sie beinhaltet eine Einspanneinrichtung mit einer Mehrzahl von starr nebeneinander angeordneten, voneinander beabstandeten Einspannstellen zum Einspannen der Garnproben. Jede Einspannstelle beinhaltet eine Garneinlauföse und zwei stromabwärts der Garneinlauföse angeordnete, fluchtende Garnklemmen. Die Garneinlauföse und die beiden Garnklemmen liegen voneinander beabstandet im Wesentlichen auf einer Einspanngeraden. Die Einspanngeraden aller Einspannstellen liegen in einer Einspannebene. Ferner beinhaltet die Garnwechselvorrichtung eine Transporteinrichtung zum Transportieren der Einspanneinrichtung, wodurch eine zu prüfende, an irgendeiner der Einspannstellen eingespannte Garnprobe in eine vorgegebene Prüfposition transportierbar ist. Die Garneinlauföse und die Garnklemmen einer jeden Einspannstelle sind derart ausgestaltet, dass die Garnprobe in einer Einlegerichtung, die im Wesentlichen senkrecht zur Einspanngeraden und senkrecht zur Einspannebene verläuft, in die Garneinlauföse und die Garnklemmen einlegbar ist.

Erfindungsgemäß beinhaltet jede Garnklemme ein Paar Klemmteller mit im Wesentlichen zueinander parallelen, zum Einklemmen der Garnprobe zusammenwirkenden Klemmflächen, welche Klemmflächen im Wesentlichen senkrecht zur Einspannebene und parallel zur Einspanngeraden liegen. Die Klemmteller bilden vorzugsweise einen entgegen der Einlegerichtung offenen Einlegeschlitz, durch den die Garnprobe zwischen die Klemmflächen führbar ist.

In einer Ausführungsform sind die Einspannstellen auf einer ebenen Trägerplatte, die parallel zur Einspannebene liegt, angeordnet. Die Trägerplatte trägt vorzugsweise die Garnklemmen. Die Trägerplatte kann einen aus der Ebene der Trägerplatte heraus ragenden Flansch aufweisen, der die Garneinlaufösen trägt.

Es ist vorteilhaft, wenn die Garneinlauföse und die beiden Garnklemmen miteinander fluchten.

In einer Ausführungsform ist die Transporteinrichtung derart ausgestaltet, dass sie eine Linearverschiebung der Einspanneinrichtung bewirkt.

Erfindungsgemäß beinhaltet die Garnwechselvorrichtung eine Öffnungseinrichtung zum aktiven Öffnen mindestens einer der beiden Garnklemmen und vorzugsweise zum aktiven, unabhängigen Öffnen beider Garnklemmen derjenigen Einspannstelle, die sich in der Prüfposition befindet. Die Öffnungseinrichtung kann einen pneumatischen Antrieb beinhalten.

In einer Ausführungsform beinhaltet jede Garnklemme ein Paar Klemmteller mit im Wesentlichen zueinander parallelen, zusammenwirkenden Klemmflächen und einen Druckmechanismus zum Beaufschlagen der Klemmteller mit einer die Klemmflächen gegeneinander drückenden Druckkraft. Der Druckmechanismus beinhaltet zwei Arme, von denen jeder einen der beiden Klemmteller trägt, ein Scharnier, welches die beiden Arme über eine Scharnierachse drehbar verbindet, und Mittel zum Ausüben eines Drehmomentes auf mindestens einen der beiden Arme.

In einer Ausführungsform ist jeder der beiden Arme der Garnklemme um eine zur Scharnierachse parallele Achse drehbar gelagert, mindestens einer der beiden Arme ist zudem senkrecht zur Scharnierachse verschiebbar gelagert und die Mittel zum Ausüben eines Drehmomentes üben eine von den Klemmtellern weg gerichtete Kraft auf das Scharnier. Die Mittel zum Ausüben eines Drehmomentes können eine vorgespannte Feder beinhalten, welche die von den Klemmtellern weg gerichtete Kraft auf das Scharnier ausübt.

In einer Ausführungsform sind die Klemmteller der Garnklemme derart auf den beiden Armen gelagert und geführt, dass im geschlossenen Zustand die beiden Klemmflächen parallel zueinander sind und eine gegenseitige Drehung der Klemmteller um eine senkrecht zur jeweiligen Klemmfläche liegende Achse unmöglich ist.

Die Öffnungseinrichtung kann bei ihrer Betätigung ein Gegendrehmoment, welches dem durch die Mittel zum Ausüben eines Drehmomentes ausgeübten Drehmoment entgegengesetzt und betragsmässig grösser als jenes ist, auf mindestens einen der beiden Arme ausüben. Die Öffnungseinrichtung weist vorzugsweise einen Stössel auf, der bei einer Betätigung der Öffnungseinrichtung entgegen der von der Feder ausgeübten Kraft auf das Scharnier drückt und dadurch die Klemmflächen voneinander entfernt.

Das erfindungsgemässe Garnprüfgerät dient zur sequenziellen Prüfung einer Mehrzahl von Garnproben. Es beinhaltet einen Garnpfad, mindestens einen entlang des Garnpfades angeordneten Sensor zur Prüfung einer Garnprobe, eine halbautomatische Garnwechselvorrichtung zur Bereitstellung der Mehrzahl von Garnproben für die Prüfung in dem Garnprüfgerät und eine automatische Einführungsvorrichtung zur Entnahme einer der in der halbautomatischen Garnwechselvorrichtung bereitgestellten Garnproben und zu ihrer Einführung in den Garnpfad. Die halbautomatische Garnwechselvorrichtung ist eine erfindungsgemässe halbautomatische Garnwechselvorrichtung wie oben beschrieben.

In einer Ausführungsform des erfindungsgemässen Garnprüfgerätes ist die Einspannebene so geneigt, dass die Garnklemmen einer jeden Einspannstelle höher liegen als die Garneinlauföse derselben Einspannstelle, und deren Neigungswinkel zur Horizontalen zwischen 0° und 45° und vorzugsweise 25° beträgt. Vorzugsweise sind die Einspannstellen horizontal nebeneinander angeordnet, und alle Garneinlaufösen liegen auf derselben Höhe. In einer Ausführungsform ist die halbautomatische Garnwechselvorrichtung auf einer Decke des Garnprüfgerätes angebracht und gegenüber einer Front des Garnprüfgerätes um mindestens einen Drittel der Tiefe des Garnprüfgerätes nach hinten versetzt.

In einer Ausführungsform ist die Transporteinrichtung der halbautomatischen Garnwechselvorrichtung derart ausgestaltet, dass sie eine Linearverschiebung der Einspanneinrichtung bewirkt und die Richtung der Linearverschiebung senkrecht zum Garnpfad liegt. Die Richtung der Linearverschiebung liegt vorzugsweise parallel zu einer durch eine Front des Garnprüfgerätes definierten Frontebene.

In einer Ausführungsform weist die automatische Einführungsvorrichtung einen Greifer zum Ergreifen der in der halbautomatischen Garnwechselvorrichtung bereitgestellten Garnprobe auf. Der Greifer ist einerseits in einer Richtung, die parallel zum Garnpfad liegt, vorzugsweise linear verschiebbar, und andererseits um eine Drehachse, die senkrecht zum Garnpfad und parallel zu einer durch eine Front des Garnprüfgerätes definierten Frontebene liegt, drehbar.

Die Erfindung erleichtert das Einlegen der Garnproben in die Garnwechselvorrichtung. Die Bedienperson kann jeweils ein Ende einer Garnprobe von einem hinter dem Garnprüfgerät befindlichen Garnwagen abziehen und mit einer einzigen Handbewegung in die Garnwechselvorrichtung einlegen. Wenn sie dies mit allen Garnproben getan hat, ist die Garnwechselvorrichtung fertig beschickt; weitere Manipulationen erübrigen sich. Dadurch spart die Bedienperson beim Beschicken der Garnwechselvorrichtung wertvolle Zeit. Ausserdem sorgen die Garnklemmen für eine optimale Einspannung der Garnproben. Jede Garnprobe befindet sich mit der richtigen Spannung an einer genau definierten Position und kann so zuverlässig von einer Einführungsvorrichtung ergriffen werden, um in den Garnpfad eingeführt zu werden. Nach der Ergreifung der Garnprobe durch die Einführungsvorrichtung werden die Garnklemmen aktiv geöffnet, wodurch die Zuverlässigkeit der Garneinführung in den Garnpfad erhöht wird.

### AUFZÄHLUNG DER ZEICHNUNGEN

Nachfolgend wird eine Ausführungsform der Erfindung anhand der Zeichnungen detailliert erläutert.
- Figur 1: zeigt ein erfindungsgemässes Garnprüfgerät in einer perspektivischen Ansicht von links vorn.
- Figur 2: zeigt eine erfindungsgemässe Garnwechselvorrichtung in einer perspektivischen Ansicht von oben rechts vorn.
- Figur 3: zeigt einen Teil der erfindungsgemässen Garnwechselvorrichtung in einer perspektivischen Ansicht.
- Figur 4: zeigt eine Garnklemme für die erfindungsgemässe Garnwechselvorrichtung in einer Explosionsansicht.
- Figur 5: zeigt perspektivische Ansichten der Garnklemme von Figur 4, und zwar (a) in geschlossenem Zustand und (b) in offenem Zustand.
- Figur 6: zeigt einen Teil der erfindungsgemässen Garnwechselvorrichtung in einem Längsschnitt entlang einem Teil der Ebene VI-VI in Figur 2.
- Figur 7: zeigt einen Teil der erfindungsgemässen Garnwechselvorrichtung in einer perspektivischen Ansicht von unten rechts.
- Figur 8: zeigt schematisch die gegenseitige Anordnung der erfindungsgemässen Garnwechselvorrichtung und einer Einführungsvorrichtung in einem erfindungsgemässen Garnprüfgerät.
- Figur 9: zeigt schematisch die gegenseitige Anordnung der Einspannebene, der Einspanngeraden und der Einlegerichtung in der erfindungsgemässen Garnwechselvorrichtung.

### AUSFÜHRUNG DER ERFINDUNG

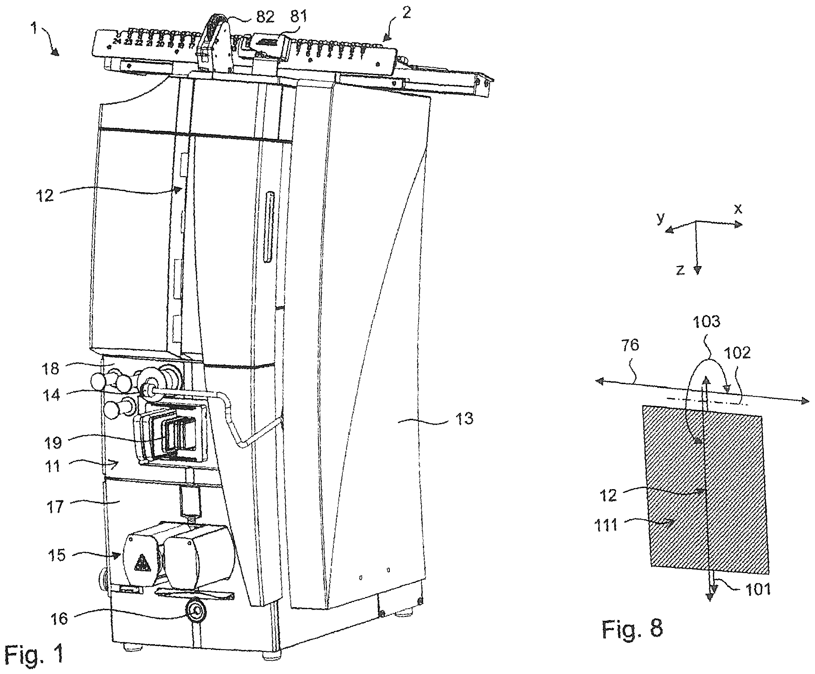

**Figur 1** zeigt beispielhaft eine Ausführungsform des erfindungsgemässen Garnprüfgerätes 1 in einer Ansicht von links vorn. Das Garnprüfgerät 1 weist eine Front 11 auf, entlang welcher ein Garnpfad 12 für eine zu prüfende (in Figur 1 nicht eingezeichnete) Garnprobe verläuft. Auf einer Seite des Garnprüfgerätes 1, welche von der Front 11 verschieden ist, ist eine automatische Einführungsvorrichtung 13 zum Einführen der Garnprobe in den Garnpfad 12 angebracht. Zum Einbringen in den Garnpfad 12 wird die Garnprobe von einem verschiebbaren und drehbaren Greifer 14 der Einführungsvorrichtung 13 ergriffen und durch eine entsprechende Bewegung des Greifers 14 eingebracht.

Während des Prüfvorgangs tritt die Garnprobe durch eine erfindungsgemässe automatische Garnwechselvorrichtung 2, auf welche unten detailliert eingegangen wird, in den Garnpfad 12 ein. Im Garnpfad 12 durchläuft die Garnprobe verschiedene Sensoren. Die Garnprobe wird von einer Fördereinrichtung 15 entlang ihrer Längsrichtung durch den Garnpfad 12 gefördert. Schliesslich verlässt die Garnprobe den Garnpfad 12 durch eine Absaugöffnung 16.

Das Garnprüfgerät 1 hat einen turmartigen Aufbau. Der turmartige Aufbau kann verschiedene Funktionsmodule umfassen. Ein erstes Funktionsmodul 17 beinhaltet im vorliegenden Fall die Fördereinrichtung 15 und die Absaugöffhung 16. Ein zweites Funktionsmodul 18 beinhaltet eine kapazitive Sensorbaugruppe 19 zur Gleichmässigkeitsprüfung des Garns. Weitere, oberhalb des zweiten Funktionsmoduls 18 befindliche Funktionsmodule können weitere, z. B. optische Sensoren beinhalten, sind jedoch wegen einer Frontabdeckung in Figur 1 nicht sichtbar.

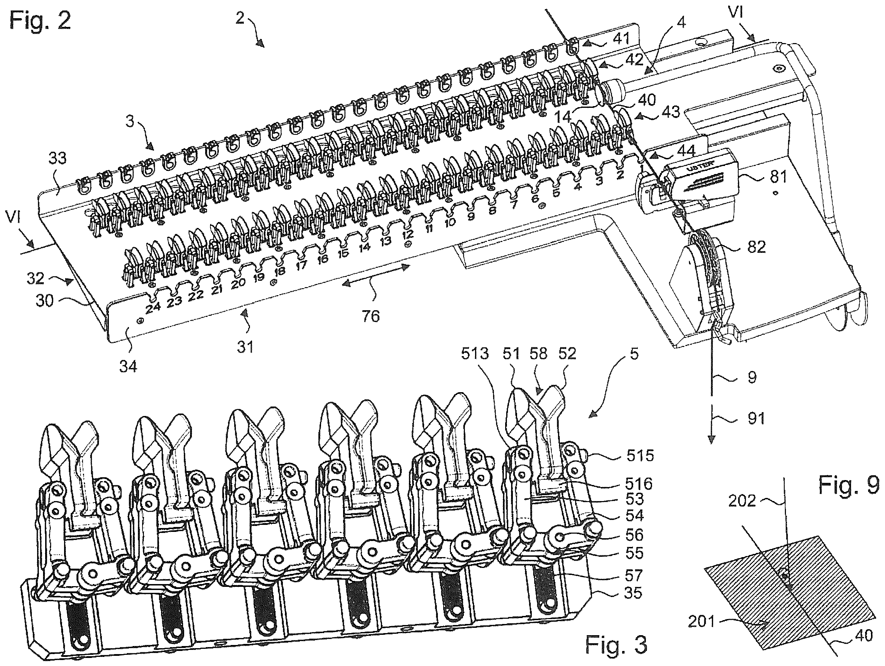

In **Figur 2** ist die erfindungsgemässe Garnwechselvorrichtung 2 in einer Ansicht von oben rechts vorn dargestellt. Sie beinhaltet eine Einspanneinrichtung 3 mit einer Mehrzahl von starr nebeneinander angeordneten, voneinander beabstandeten Einspannstellen 4 zum Einspannen von Garnproben 9. Die hier diskutierte Ausführungsform hat 24 Einspannstellen 4, die äquidistant auf einer Länge von 455 mm angeordnet sind; die Äquidistanz beträgt somit 19.8 mm. Jede Einspannstelle 4 beinhaltet eine Garneinlauföse 41 und zwei stromabwärts der Garneinlauföse 41 angeordnete, fluchtende Garnklemmen 42, 43. Die Garneinlauföse 41 und die beiden Garnklemmen 42, 43 liegen voneinander beabstandet im Wesentlichen auf einer Einspanngeraden 40. Die beiden Garnklemmen 42, 43 dienen zum Einspannen der Garnprobe 9. Die Garneinlauföse 41 dient dazu, eine Grobposition für die Garnprobe 9 vorzugeben und eine erste Garnklemme 42 von Zugkräften quer zur Garnlängsrichtung zu entlasten. Ferner kann jede Einspannstelle 4 eine stromabwärts der beiden Garnklemmen 42, 43 angeordnete Garnauslauföse 44 beinhalten. Die Garnauslauföse 44 liegt aber vorzugsweise unterhalb der Einspanngeraden 40 und soll nicht mit der laufenden Garnprobe 9 wechselwirken; sie dient vielmehr bloss dazu, im Ruhezustand das freie Ende einer in der Einspannstelle 4 eingespannten Garnprobe 9 in einer definierten Position zu halten. Entlang der Einspanngeraden 40 gemessen, können Entfernungen zwischen Mitten der vier Bauteile 41-44 die folgenden Werte annehmen: Garneinlauföse 41 - erste Garnklemme 42: zwischen 30 mm und 50 mm und vorzugsweise 40 mm; erste Garnklemme 42 - zweite Garnklemme 43: zwischen 50 mm und 90 mm und vorzugsweise 70 mm; zweite Garnklemme 43 - Garnauslauföse 44: zwischen 30 mm und 50 mm und vorzugsweise 40 mm.

In der hier diskutierten Ausführungsform sind die Einspannstellen 4 auf einer ebenen, im Wesentlichen rechteckigen Trägerplatte 30 angeordnet. Eine längere Seite 31 der Trägerplatte 30 verläuft horizontal. Eine kürzere Seite 32 der Trägerplatte 30 ist parallel zur Einspanngeraden 40 und bspw. um 25° gegen die Horizontale so geneigt, dass die Garnlaufrichtung schräg von unten nach oben verläuft. Bei einer solchen Neigung liegen die Garnklemmen 42, 43 einer jeden Einspannstelle 4 höher als die Garneilauföse 41 derselben Einspannstelle 4. Die Einspannstellen 4 sind horizontal nebeneinander angeordnet, und alle Garneinlaufösen 41 liegen auf derselben Höhe. Die Neigung der Trägerplatte 30 gegen die Rückseite des Garnprüfgerätes 1 hin nach unten erleichtert das Einlegen der Garnproben 9 in die Einspanneinrichtung 4. Um von dieser Erleichterung Gebrauch zu machen, sollte der Neigungswinkel der Trägerplatte 30 gegen die Horizontale zwischen 0° und 45° betragen. Die Trägerplatte 30 kann auch horizontal liegen (Neigungswinkel 0°), sollte aber nicht nach vorn geneigt sein, weil dadurch das Einlegen erschwert würde. Die Trägerplatte 30 trägt die Garnklemmen 42, 43. Sie weist an ihren stromaufwärts und stromabwärts gelegenen Rändern aus ihrer Ebene heraus ragende Flansche 33, 34 auf, welche die Garneinlaufösen 41 bzw. die Garnauslaufösen 44 tragen.

In Figur 2 ist in einer ersten Einspannstelle 4 eine Garnprobe 9 eingezeichnet. Die Längsrichtung des in der Einspannstelle befindlichen Teils der Garnprobe 9 fällt mit der Einspanngeraden 40 zusammen. Die Laufrichtung der Garnprobe 9 während ihrer Prüfung in Prüfgerät 1 ist durch einen Pfeil 91 angedeutet, auf welche Laufrichtung sich auch die in dieser Schrift verwendeten Begriffe "stromabwärts" und "stromaufwärts" beziehen.

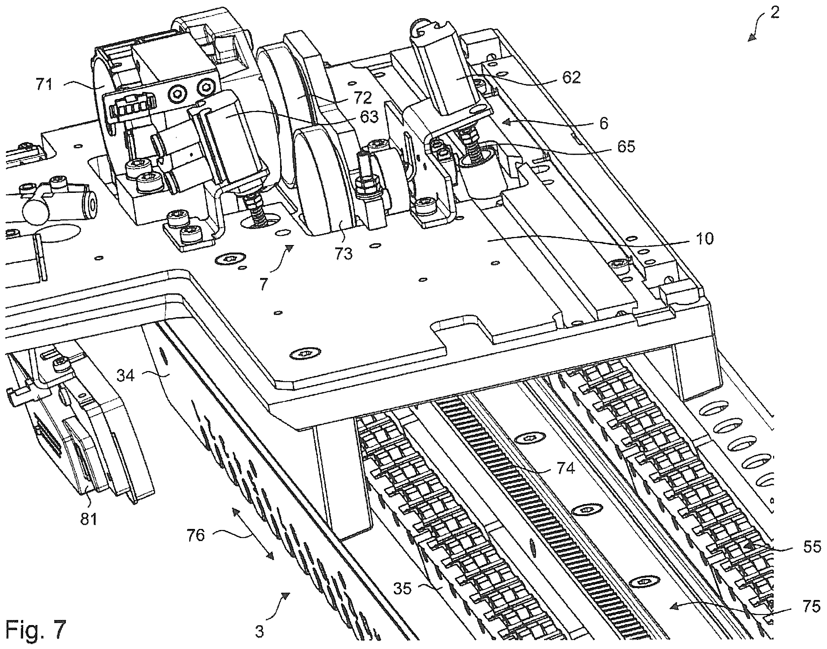

Ferner beinhaltet die erfindungsgemässe Garnwechselvorrichtung 2 eine Transporteinrichtung 7 zum Transportieren der Einspanneinrichtung 3. Die Transporteinrichtung 7 ist in Figur 2 nicht sichtbar und wird weiter unten mit Bezug auf Figur 7 beschrieben. In der hier diskutierten Ausführungsform handelt es sich beim Transport der Einspanneinrichtung 3 um eine Linearverschiebung. Die Verschiebungsrichtung ist durch einen Doppelpfeil 76 angedeutet. Sie ist horizontal, parallel zur längeren Seite 31 der Trägerplatte 30 und senkrecht zur Einspanngeraden 40. Das Transportieren der Einspanneinrichtung 3 dient dazu, eine zu prüfende, an irgendeiner der Einspannstellen 4 eingespannte Garnprobe 9 in eine Prüfposition zu bringen. Die Prüfposition ist durch die Lage des Garnpfades 12 im Garnprüfgerät 1 vorgegeben. Die in Figur 2 eingezeichnete Garnprobe 9 befindet sich gerade in der Prüfposition. Ein mit dem Garnpfad 12 fluchtendes Umlenkrad 82 kann die in der Prüfposition befindliche Garnprobe 9 zum Garnpfad 12 hin umlenken. Zwischen der Einspanneinrichtung 3 und dem Umlenkrad 82 kann sich eine Schneideinrichtung 81 zum Durchtrennen der Garnprobe 9 nach erfolgter Prüfung befinden.

In Figur 2 ist auch der Greifer 14 der Einführungsvorrichtung 13 eingezeichnet, und zwar in einer Position, in welcher er die in der Prüfposition befindliche Garnprobe 9 ergreift. Das Ergreifen erfolgt zwischen den beiden Garnklemmen 42, 43. Dort ist die Position der Garnprobe 9 am besten definiert, und die Garnprobe 9 ist dort straff gespannt, während sie von den beiden Garnklemmen 42, 43 eingeklemmt ist. Nachdem der Greifer 14 die Garnprobe 9 ergriffen hat, werden die beiden Garnklemmen 42, 43 aktiv geöffnet, und die Einführungsvorrichtung 13 führt die ergriffene Garnprobe 9 über das Umlenkrad 82 bis zur Absaugöffnung 16 hin in den Garnpfad 12 ein.

Wie aus den Figuren 1 und 2 ersichtlich, ist die Garnwechselvorrichtung 2 auf einer Decke des Garnprüfgerätes 1 angebracht und gegenüber der Front 11 des Garnprüfgerätes 1 um mindestens einen Drittel und vorzugsweise um mehr als die Hälfte der Tiefe des Garnprüfgerätes 1 nach hinten versetzt. Dies erleichtert das Einlegen der Garnproben 9 von hinten.

In der hier diskutierten Ausführungsform der Erfindung sind alle Garnklemmen 42, 43 der Einspanneinrichtung 3 identisch und können daher mit einem einzigen Bezugszeichen 5 bezeichnet werden. Die **Figuren 3-5** zeigen den Aufbau einer Ausführungsform einer Garnklemme 5, die für den Einsatz in der erfindungsgemässen Garnwechselvorrichtung 2 besonders geeignet ist. Die Garnklemme 5 beinhaltet ein Paar Klemmteller 51, 52 mit im Wesentlichen zueinander parallelen, zum Einklemmen der (in Figur 2 sichtbaren, in Figuren 3-5 nicht eingezeichneten) Garnprobe 9 zusammenwirkenden Klemmflächen 511, 521. Die oberen Enden der Klemmteller 51, 52 bilden zusammen einen Einlegeschlitz 58, der einen im Wesentlichen V-förmigem Querschnitt aufweist, nach oben offen ist und sich zu den Klemmflächen 511, 521 hin verengt. Der Einlegeschlitz 58 führt die Garnprobe 9 beim Einlegen zwischen die Klemmflächen 511, 521. Eine Klemmfläche 511 der zusammenwirkenden Klemmflächen 511, 521 weist an ihrem stromaufwärts gelegenen Teil eine Einlaufrille 512 auf. Die Einlaufrille 512 nimmt die Garnprobe 9 auf und verhindert ihr Herausrutschen aus der Garnklemme 5 bei auf die Garnprobe 9 wirkendem Zug in Laufrichtung 91.

Die Garnklemme 5 weist ferner ein Scharnier 55 auf, das zwei Arme 53, 54 um eine Scharnierachse 56 drehbar miteinander verbindet. Als Scharnierachse dient ein Stift 56. Jeder der beiden Arme 53, 54 ist im Wesentlichen rechtwinklig gewinkelt und trägt an seinem vom Scharnier 55 abgewandten Ende einen der beiden Klemmteller 51, 52. Bei Drehung der Arme 53, 54 um die Scharnierachse 56 werden die beiden Klemmteller 51, 52 gegeneinander bzw. voneinander weg bewegt. Jeder der Klemmteller 51, 52 ist mittels eines Kugelgelenks auf dem entsprechenden Arm 53, 54 gelagert. Diese drehbare Lagerung stellt sicher, dass im geschlossenen Zustand die beiden Klemmflächen 511, 521 parallel zueinander sind. Das Kugelgelenk besteht aus einer auf dem Klemmteller 51, 52 befestigten Kugel 513, 523 und zwei in dem Arm 53, 54 geformten Pfannen 514, 524, die derart voneinander beabstandet sind, dass sie die Kugel 513, 523 klemmend aufnehmen können.

Ein Klemmteller 51 der beiden Klemmteller 51, 52 trägt einen Zapfen 515, der in einer entsprechenden Öffnung 525 im anderen Klemmteller 52 geführt wird. Dieser die Garnklemme 5 durchquerende Zapfen 515 hat zwei Aufgaben. Erstens definiert er die Höhenposition der einzuklemmenden bzw. eingeklemmten Garnprobe 9. Zweitens stellt er sicher, dass sich die beiden in ihren Kugelgelenken gelagerten Klemmteller 51, 52 nicht zu stark gegeneinander verdrehen können, sondern die beiden Klemmflächen 511, 521 immer annähernd parallel zueinander bleiben.

An ihren unteren Enden tragen die Klemmteller 51, 52 einen bzw. zwei Finger 516, 526. Diese Finger 516, 526 greifen ineinander und wirken so zusammen, dass eine gegenseitige Drehung der Klemmteller 51, 52 um eine durch den Zapfen 515 definierte Achse unmöglich ist.

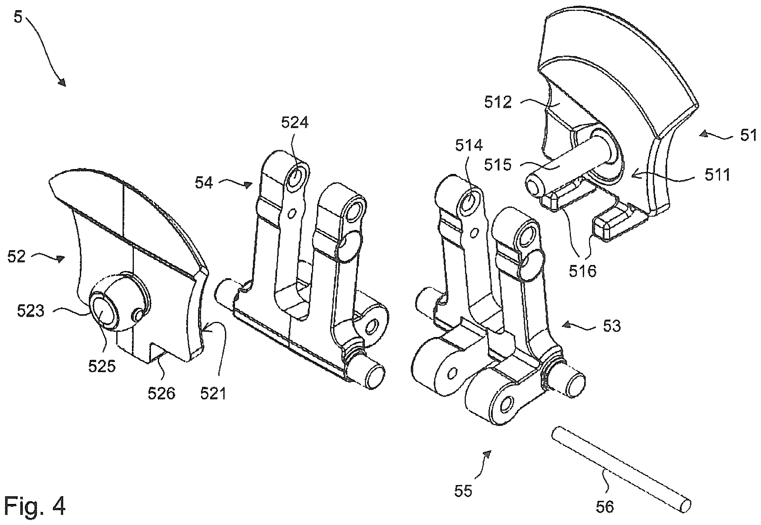

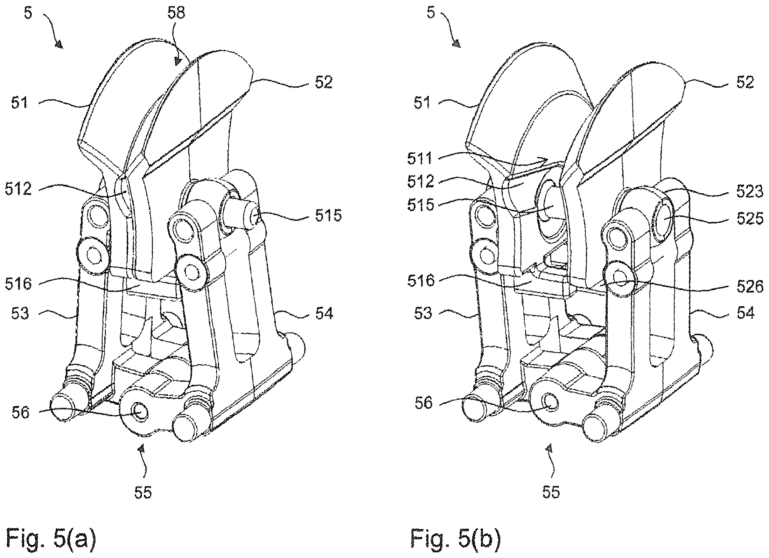

Alle in den Figuren 4 und 5 dargestellten Teile der Garnklemme 5 sind vorzugsweise durch Spritzgiessen aus einem Kunststoff hergestellt. Die Montage der Garnklemme 5 erfolgt durch einfaches Zusammenstecken der fünf Teile 51-54, 56.

Ferner beinhaltet die Garnklemme 5 einen Druckmechanismus zum Beaufschlagen der Klemmteller 51, 52 mit einer die Klemmflächen 511, 521 gegeneinander drückenden Druckkraft. In der hier diskutierten Ausführungsform beinhaltet der Druckmechanismus die beiden Arme 53, 54, das Scharnier 55 und eine vorgespannte Zugfeder 57 (siehe **Figur 3**), die einerseits mit dem Stift 56 des Scharniers 55 und andererseits mit einer Halterung 35 verbunden ist. Jeder der beiden Arme 53, 54 ist um eine zur Scharnierachse 56 parallele Achse drehbar in der Halterung 35 gelagert. Ein Arm 53 der beiden Arme 53, 54 ist zudem senkrecht zur Scharnierachse 56 verschiebbar in der Halterung 35 gelagert, um eine Drehung der beiden Arme 53, 54 um die Scharnierachse 56 zu ermöglichen. Mit ihrer Vorspannung zieht die Zugfeder 57 das Scharnier 55 ständig nach unten, von den Klemmtellern 51, 52 weg. Dadurch übt die Zugfeder 57 zusammen mit der Halterung 35 ein Drehmoment auf jeden der beiden Arme 53, 54 aus. Durch diese Drehmomente werden die Klemmteller 51, 52 mit ihren Klemmflächen 511, 521 gegeneinander gedrückt.

Die Halterung 35 ist von unten an der Trägerplatte 30 befestigt. Ein Paar solcher Halterungen 35 kann z. B. sechs Garnklemmen 5 aufnehmen, so dass für die Garnwechselvorrichtung 2 gemäss Figur 2 acht Paare von Halterungen 35 benötigt werden. Die Trägerplatte 30 weist entsprechende Durchgangsöffnungen für die Garnklemmen 5 auf, welche die Scharniere 55 von unten zugänglich machen.

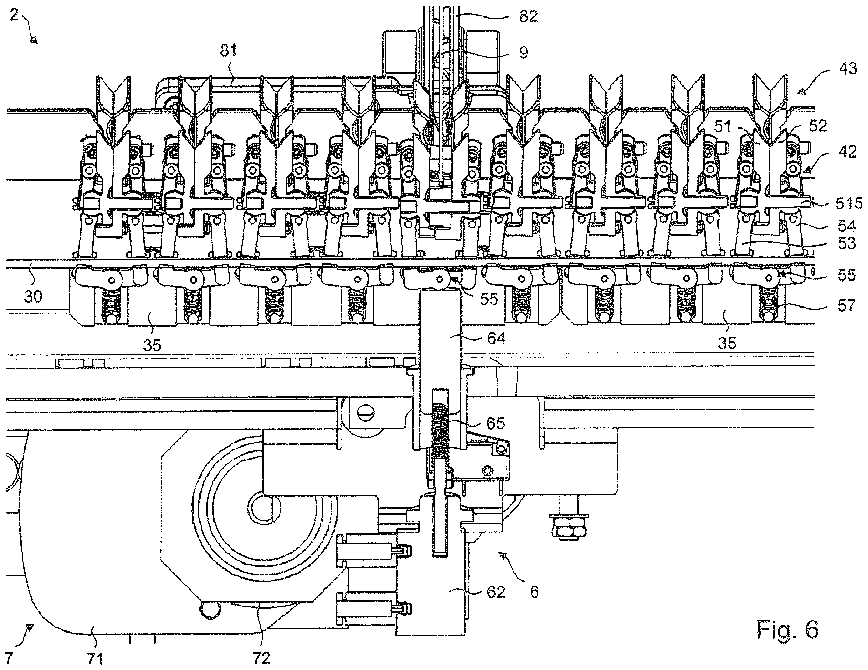

Um die Garnklemme 5 zu öffnen, ist die erfindungsgemässe Garnwechselvorrichtung 2 mit einer aktiven Öffnungseinrichtung 6 ausgestattet, die anhand der **Figuren 6** **und** **7** erläutert wird. Die Öffnungseinrichtung 6 ist unter der Trägerplatte 30 unterhalb der Prüfposition angebracht. Sie beinhaltet einen Stössel 64, der bei Betätigung der Öffnungseinrichtung 6 von unten auf das Scharnier 55 der Garnklemme 5 drückt. Der Druck des Stössels 64 wirkt der Zugkraft der Zugfeder 57 entgegen und ist stärker als jene. Somit übt der Stössel 64 zusammen mit der Halterung 35 ein Gegendrehmoment auf jeden der beiden Arme 53, 54 aus, welches dem von der Zugfeder 57 ausgeübten Drehmoment entgegengesetzt und betragsmässig grösser ist als jenes. Das Gegendrehmoment entfernt die beiden Klemmteller 51, 52 voneinander, öffnet also die Garnklemme 5. Dementsprechend sind in Figur 6 die beiden Garnklemmen 42, 43, die sich in der Prüfposition befinden, im offenen Zustand eingezeichnet. Zum Schliessen der Garnklemme 5 wird der Stössel 64 zurückgezogen, wonach die Wirkung der Zugfeder 57 wieder einsetzt und die beiden Klemmteller 51, 52 gegeneinander drückt. Der Stössel 64 wird über eine Entlastungsfeder 65 von einem pneumatischen Antriebszylinder 62 angetrieben. Für jede der beiden Garnklemmen 42, 43 sind ein eigener pneumatischer Antriebszylinder 62, 63 (siehe Figur 7) und ein eigener Stössel vorgesehen. Die Antriebszylinder 62, 63 können einzeln und unabhängig voneinander angesteuert werden, so dass die beiden Garnklemmen 42, 43 einzeln und unabhängig voneinander geöffnet und geschlossen werden können.

Anhand der **Figur 7** wird hauptsächlich die Transporteinrichtung 7 der erfindungsgemässen Garnwechselvorrichtung 2 erläutert. Die Transporteinrichtung 7 dient zur Hin- und Herverschiebung 76 der Einspanneinrichtung 3. In der Ansicht von unten, wie sie in Figur 7 dargestellt ist, sieht man eine Zahnstange 74, die auf einer Unterseite der Trägerplatte 30 parallel zu ihrer längeren Seite 31 befestigt ist. Auf einer Deckplatte 10, die fest auf einem Rahmengehäuse des Garnprüfgerätes 1 montiert ist, ist ein von einem Motor 71 über ein Umsetzzahnrad 72 antreibbares Zahnrad 73 drehbar gelagert, das mit der Zahnstange 74 in Eingriff steht. Statt des Umsetzzahnrades 72 könnte irgendein an sich bekanntes Getriebe verwendet werden. Die Trägerplatte 30 ist relativ zum Rahmengehäuse des Prüfgerätes 1 in einer Linearführung 75 verschiebbar. Der Motor 71, das Umsetzzahnrad 72, das Zahnrad 73, die Zahnstange 74 und die Linearführung 75 sind Bestandteile der Transporteinrichtung 7. Die Transporteinrichtung 7 kann automatisch angesteuert werden, bspw. von einer (nicht eingezeichneten) Steuereinheit, die sich im Garnprüfgerät 1 oder ausserhalb desselben befinden kann. Alternativ oder zusätzlich kann die Transporteinrichtung 7 manuell angesteuert werden. Für die letztere Variante kann ein (nicht eingezeichneter) Taster an der Transporteinrichtung 7 oder an einem anderen Teil des Garnprüfgerätes 1 angebracht sein, dessen Betätigung eine Verschiebung der Einspanneinrichtung 3 um eine oder mehrere Einspannstellen auslöst.

**Figur 8** zeigt schematisch die gegenseitige Anordnung der erfindungsgemässen Garnwechselvorrichtung 2 und der Einführungsvorrichtung 13 im erfindungsgemässen Garnprüfgerät 1; zum besseren Verständnis wird empfohlen, diese schematische Zeichnung zusammen mit der perspektivischen Ansicht von Figur 1 zu betrachten. Der hier als gerade angenommene Garnpfad 12 verläuft im Wesentlichen senkrecht nach unten in einer Richtung z. Die Front 11 des Garnprüfgerätes 1 definiert eine Frontebene 111, die durch die Achsen x und z aufgespannt wird und die den Garnpfad 12 enthält. Die Verschiebungsrichtung 76 der Einspanneinrichtung 4 liegt parallel zur Richtung x, d. h. senkrecht zum Garnpfad 12 und parallel zur Frontebene 111. Der Greifer 14 der Einführungsvorrichtung 13 ist einerseits in einer Richtung 101, die parallel zum Garnpfad 12 und zur Richtung z liegt, verschiebbar. Die Verschiebung ist im Wesentlichen linear, bis auf eine mögliche leichte Krümmung des Garnpfades 12. Andererseits ist der Greifer 14 der Einführungsvorrichtung 13 um eine Drehachse 102, die senkrecht zum Garnpfad 12 und parallel zur Frontebene 111 liegt, drehbar, was mit einem Doppelpfeil 103 angedeutet ist. Man kann auch sagen, dass die Drehachse 102 parallel zur Verschiebungsrichtung 76 der Einspanneinrichtung 4 und zur Richtung x liegt. Die Drehebene, in welcher die Drehung 103 des Greifers 14 stattfindet, wird somit von den Achsen y und z aufgespannt und steht senkrecht auf der Frontebene 111.

**Figur 9** zeigt schematisch die gegenseitige Anordnung einer Einspannebene 201, der Einspanngeraden 40 und einer Einlegerichtung 202 in der erfindungsgemässen Garnwechselvorrichtung 2; zum besseren Verständnis wird empfohlen, diese schematische Zeichnung zusammen mit der perspektivischen Ansicht von Figur 2 zu betrachten. Die Einspannebene 201 ist diejenige Ebene, in der die Einspanngeraden 40 aller Einspannstellen 4 (siehe Figur 2) liegen. Die Einspannebene 201 liegt somit parallel zur ebenen Trägerplatte 30 der Ausführungsform von Figur 2. Die Garnprobe 9 wird in der Einlegerichtung 202 in die Garneinlauföse 41 und die Garnklemmen 42, 43 eingelegt. Gemäss der vorliegenden Erfindung sind die Garneinlauföse 41 und die Garnklemmen 42, 43 einer jeden Einspannstelle 4 derart ausgestaltet, dass die Einlegerichtung 202 im Wesentlichen senkrecht zur Einspanngeraden 40 und senkrecht zur Einspannebene 201 verläuft. Dies vereinfacht das manuelle Einlegen der Garnproben 9.

Nachfolgend wird die Funktionsweise der erfindungsgemässen Garnwechselvorrichtung 2 beschrieben.

Vor der Prüfung der Garnproben 9 durch das Prüfgerät 1 muss die Garnwechselvorrichtung 2 beschickt werden. Dabei befindet sich die Bedienperson hinter dem Garnprüfgerät 1, d. h. sie hat im Wesentlichen eine Rückseite des Garnprüfgerätes 1 vor sich, die der Front 11 gegenüber liegt. Beim Beschicken sind alle Garnklemmen 42, 43 geschlossen. Die Bedienperson zieht jeweils ein Ende einer Garnprobe 9 von einem hinter dem Garnprüfgerät 1 befindlichen Garnwagen ab und legt es mit einer einzigen Handbewegung in die Garnwechselvorrichtung 2 ein. Dieses einfache und Zeit sparende Einlegen wird erfindungsgemäss dadurch ermöglicht, dass die Garneinlauföse 41 und die beiden Garnklemmen 42, 43 auf einer Einspanngeraden 40 liegen und dass die Einlegerichtung 202 senkrecht zur Einspanngeraden 40 und senkrecht zur Einspannebene 201 verläuft. Die Klemmflächen 511, 521 der Garnklemmen 5 liegen senkrecht zur Einspannebene 201 und parallel zur Einspanngeraden 40, was das Einlegen der Garnprobe 9 in der genannten Einlegerichtung 202 ermöglicht. Ausserdem erleichtert der Einlegeschlitz 58 am oberen Ende der Garnklemme 5 das Einlegen von oben, d. h. in der Einlegerichtung 202.

Die Prüfung der Garnproben durch das Prüfgerät 1 kann beginnen, wenn alle zu prüfenden Garnproben 9 in den Einspannstellen 4 eingespannt sind. Im Ruhezustand sind die in der Prüfposition befindlichen Garnklemmen 42, 43 geschlossen, und eine zuvor eingelegte Garnprobe 9 ist zwischen den Garnklemmen 42, 43 eingespannt. Nachdem der Greifer 14 die in der Prüfposition eingespannte Garnprobe 9 ergriffen hat, wird die Öffnungseinrichtung 6 betätigt, d. h. die beiden pneumatischen Antriebszylinder 62, 63 werden so mit Druckluft beaufschlagt, dass die beiden Stössel 64 auf die entsprechenden Scharniere 55 der beiden Garnklemmen 42, 43 drücken. Dadurch öffnen sich die beiden Garnklemmen 42, 43, und die Garnprobe 9 kann von der Einführungsvorrichtung 13 aus der Einspannstelle 4 entnommen werden. Die Entnahme der Garnprobe 9 erfolgt im Wesentlichen in einer Richtung, welche der Einlegerichtung 202 entgegengesetzt ist. Nach der Entnahme aus der Einspannstelle 4 führt die Einführungsvorrichtung 13 die Garnprobe 9 in den Garnpfad 12 ein. Zu diesem Zweck wird der Greifer 14, der die Garnprobe 9 hält, um ca. 100° nach vorn gedreht, um ca. 60 cm nach unten (in Richtung +z) verschoben, und um weitere ca. 140° nach unten gedreht. Das Ende der Garnprobe 9 befindet sich dann im Bereich der Absaugöffnung 16. Die Garnprobe 9 wird vom Greifer 14 losgelassen und in die Absaugöffnung 16 hinein abgesaugt. Der Greifer 14 wird zurück gedreht und nach oben (in Richtung -z) verschoben. Im Betriebszustand, während die Garnprobe 9 von der Fördereinrichtung 15 durch den Garnpfad 12 gefördert und von den Sensoren 19 geprüft wird, müssen die beiden Garnklemmen 42, 43 offen bleiben, um den freien Lauf der Garnprobe 9 durch die Einspannstelle 4 nicht zu behindern.

Nach Beendigung der Prüfung wird die Fördereinrichtung 15 abgestellt, und die Garnprobe 9 steht still. Nun werden die Garnklemmen 42, 43 wieder geschlossen, so dass sie die Garnprobe 9 erneut einspannen. Die Schneideinrichtung 81 wird betätigt und schneidet die Garnprobe 9 stromabwärts der Einspannstelle 4 durch, wobei der Greifer 14 die Garnprobe 9 nach unten, in die Schneideinrichtung 81 hinein, drückt. Danach verschiebt die Transporteinrichtung 7 die Einspanneinrichtung 3 so weit, bis sich die nächste zu prüfende Garnprobe an der Prüfposition befindet; alternativ kann dieselbe Garnprobe 9 ohne Verschiebung der Einspanneinrichtung 3 nochmals geprüft werden. Sobald sich die nächste zu prüfende Garnprobe in der Prüfposition befindet, ist die Garnwechselvorrichtung 2 bereit für einen neuen Prüfzyklus.

### BEZUGSZEICHENLISTE

- 1: Prüfgerät
- 10: Deckplatte
- 11: Front
- 12: Garnpfad
- 13: Einführungsvorrichtung
- 14: Greifer
- 15: Fördereinrichtung
- 16: Absaugöffnung
- 17, 18: Funktionsmodule
- 19: kapazitive Sensorbaugruppe

- 2: Garnwechselvorrichtung

- 3: Einspanneinrichtung
- 30: Trägerplatte
- 31: längere Seite der Trägerplatte
- 32: kürzere Seite der Trägerplatte
- 33, 34: Flansche an der Trägerplatte
- 35: Halterung für Garnklemmen

- 4: Einspannstelle
- 40: Einspanngerade
- 41: Garneinlauföse
- 42, 43: Garnklemmen
- 44: Garnauslauföse

- 5: Garnklemme
- 51, 52: Klemmteller
- 511, 521: Klemmflächen
- 513, 523: Kugeln der Kugelgelenke
- 514, 524: Pfannen der Kugelgelenke
- 515: Zapfen
- 525: Öffnung
- 516, 526: Finger
- 53, 54: Arme
- 55: Scharnier
- 56: Scharnierachse, Stift
- 57: Zugfeder
- 58: Einlegeschlitz

- 6: Öffnungseinrichtung
- 62, 63: pneumatische Antriebszylinder
- 64: Stössel
- 65: Entlastungsfeder

- 7: Transporteinrichtung
- 71: Motor
- 72: Umsetzzahnrad
- 73: Zahnrad
- 74: Zahnstange
- 76: Verschiebungsrichtung der Einspanneinrichtung

- 81: Schneideinrichtung
- 82: Umlenkrad

- 9: Garnprobe
- 91: Laufrichtung der Garnprobe

- 101: Verschiebungsrichtung der Einführungsvorrichtung
- 102: Drehachse der Einführungsvorrichtung
- 103: Drehung der Einführungsvorrichtung
- 111: Frontebene

- 201: Einspannebene
- 202: Einlegerichtung

## Patentansprüche

1. Halbautomatische Garnwechselvorrichtung (2) zur Bereitstellung einer Mehrzahl von Garnproben (9) für die Prüfung in einem Garnprüfgerät (1), beinhaltend eine Einspanneinrichtung (3) mit einer Mehrzahl von starr nebeneinander angeordneten, voneinander beabstandeten Einspannstellen (4) zum Einspannen der Garnproben (9), wobei
jede Einspannstelle (4) eine Garneinlauföse (41) und zwei stromabwärts der Garneinlauföse (41) angeordnete, fluchtende Garnklemmen (42, 43) beinhaltet, die Garneinlauföse (41) und die beiden Garnklemmen (42, 43) in jeder Einspannstelle (4) voneinander beabstandet im Wesentlichen auf einer Einspanngeraden (40) liegen und
die Einspanngeraden (40) aller Einspannstellen (4) in einer Einspannebene (201) liegen, und
eine Transporteinrichtung (7) zum Transportieren der Einspanneinrichtung (3), wodurch eine zu prüfende, an irgendeiner der Einspannstellen (4) eingespannte Garnprobe (9) in eine vorgegebene Prüfposition transportierbar ist,
**dadurch gekennzeichnet, dass**
die Garneinlauföse (41) und die Garnklemmen (42, 43) einer jeden Einspannstelle (4) derart ausgestaltet sind, dass die Garnprobe (9) in einer Einlegerichtung (202), die im Wesentlichen senkrecht zur Einspanngeraden (40) und senkrecht zur Einspannebene (201) verläuft, in die Garneinlauföse (41) und die Garnklemmen (42, 43) einlegbar ist,
jede Garnklemme (42, 43) ein Paar Klemmteller (51, 52) mit im Wesentlichen zueinander parallelen, zum Einklemmen der Garnprobe (9) zusammenwirkenden Klemmflächen (511, 521) beinhaltet, welche Klemmflächen (511, 521) im Wesentlichen senkrecht zur Einspannebene (201) und parallel zur Einspanngeraden (40) liegen, und
die Garnwechselvorrichtung (2) eine Öffnungseinrichtung (6) zum aktiven Öffnen mindestens einer der beiden Garnklemmen (42, 43) beinhaltet.

2. Halbautomatische Garnwechselvorrichtung (2) nach Anspruch 1, wobei die Klemmteller (51, 52) einen entgegen der Einlegerichtung (202) offenen Einlegeschlitz (58) bilden, durch den die Garnprobe (9) zwischen die Klemmflächen (511, 521) führbar ist.

3. Halbautomatische Garnwechselvorrichtung (2) nach einem der vorangehenden Ansprüche, wobei die Einspannstellen (4) auf einer ebenen Trägerplatte (30), die parallel zur Einspannebene (201) liegt, angeordnet sind.

4. Halbautomatische Garnwechselvorrichtung (2) nach Anspruch 3, wobei die Trägerplatte (30) die Garnklemmen (42, 43) trägt und einen aus der Ebene der Trägerplatte (30) heraus ragenden Flansch (33) aufweist, der die Garneinlaufösen (41) trägt.

5. Halbautomatische Garnwechselvorrichtung (2) nach einem der vorangehenden Ansprüche, wobei die Garneinlauföse (41) und die beiden Garnklemmen (42, 43) in jeder Einspannstelle (4) miteinander fluchten.

6. Halbautomatische Garnwechselvorrichtung (2) nach einem der vorangehenden Ansprüche, wobei die Transporteinrichtung (7) derart ausgestaltet ist, dass sie eine Linearverschiebung der Einspanneinrichtung (4) bewirkt.

7. Halbautomatische Garnwechselvorrichtung (2) nach einem der vorangehenden Ansprüche, wobei die Öffnungseinrichtung (6) zum aktiven, unabhängigen Öffnen beider Garnklemmen (42, 43) derjenigen Einspannstelle (4), die sich in der Prüfposition befindet, ausgebildet ist.

8. Halbautomatische Garnwechselvorrichtung (2) nach einem der vorangehenden Ansprüche, wobei jede Garnklemme (42, 43, 5) einen Druckmechanismus (53-57) zum Beaufschlagen der Klemmteller (51, 52) mit einer die Klemmflächen (511, 521) gegeneinander drückenden Druckkraft, welcher Druckmechanismus (53-57)
zwei Arme (53, 54), von denen jeder einen der beiden Klemmteller (51, 52) trägt,
ein Scharnier (55), welches die beiden Arme über eine Scharnierachse (56) drehbar verbindet, und
Mittel (57) zum Ausüben eines Drehmomentes auf mindestens einen der beiden Arme (53, 54)
aufweist,
beinhaltet.

9. Garnprüfgerät (1) zur sequenziellen Prüfung einer Mehrzahl von Garnproben (9), mit einem Garnpfad (12),
mindestens einem entlang des Garnpfades (12) angeordneten Sensor (19) zur Prüfung einer Garnprobe (9),
einer halbautomatischen Garnwechselvorrichtung (2) zur Bereitstellung der Mehrzahl von Garnproben (9) für die Prüfung in dem Garnprüfgerät (1) und
einer automatischen Einführungsvorrichtung (13) zur Entnahme einer der in der halbautomatischen Garnwechselvorrichtung (2) bereitgestellten Garnproben (9) und zu ihrer Einführung in den Garnpfad (12),
**dadurch gekennzeichnet, dass**
die halbautomatische Garnwechselvorrichtung (2) eine halbautomatische Garnwechselvorrichtung nach einem der vorangehenden Ansprüche ist.

10. Garnprüfgerät (1) nach Anspruch 9, wobei die Einspannebene (201) so geneigt ist, dass die Garnklemmen (42, 43) einer jeden Einspannstelle (4) höher liegen als die Garneinlauföse (41) derselben Einspannstelle (4), und deren Neigungswinkel zur Horizontalen zwischen 0° und 45° und vorzugsweise 25° beträgt.

11. Garnprüfgerät (1) nach Anspruch 10, wobei die Einspannstellen (4) horizontal nebeneinander angeordnet sind und alle Garneinlaufösen (41) auf derselben Höhe liegen.

12. Garnprüfgerät (1) nach einem der Ansprüche 9-11, wobei die halbautomatische Garnwechselvorrichtung (2) auf einer Decke des Garnprüfgerätes (1) angebracht und gegenüber einer Front (11) des Garnprüfgerätes (1) um mindestens einen Drittel der Tiefe des Garnprüfgerätes (1) nach hinten versetzt ist.

13. Garnprüfgerät (1) nach einem der Ansprüche 9-12, wobei die Transporteinrichtung (7) der halbautomatischen Garnwechselvorrichtung (2) derart ausgestaltet ist, dass sie eine Linearverschiebung der Einspanneinrichtung (4) bewirkt und die Richtung (76) der Linearverschiebung senkrecht zum Garnpfad (12) liegt.

14. Garnprüfgerät (1) nach Anspruch 13, wobei die Richtung (76) der Linearverschiebung parallel zu einer durch eine Front (11) des Garnprüfgerätes (1) definierten Frontebene (111) liegt.

15. Garnprüfgerät (1) nach einem der Ansprüche 9-14, wobei die automatische Einführungsvorrichtung (13) einen Greifer (14) zum Ergreifen der in der halbautomatischen Garnwechselvorrichtung (2) bereitgestellten Garnprobe (9) aufweist, und der Greifer (14) einerseits in einer Richtung (101), die parallel zum Garnpfad (12) liegt, verschiebbar, und andererseits um eine Drehachse (102), die senkrecht zum Garnpfad (12) und parallel zu einer durch eine Front (11) des Garnprüfgerätes (1) definierten Frontebene (111) liegt, drehbar ist.

## Claims

1. A semiautomatic yarn-changing device (2) for providing a plurality of yarn samples (9) for testing in a yarn-testing device (1), containing
a clamping device (3) with a plurality of mutually spaced clamping locations (4) which are rigidly arranged adjacent to each other for clamping the yarn samples (9), wherein
each clamping location (4) contains a yarn inlet eyelet (41) and two aligned yarn clamps (42, 43) which are arranged downstream of the yarn inlet eyelet (41), the yarn inlet eyelet (41) and the two yarn clamps (42, 43) lie substantially on a straight clamping line (40) in each clamping location (4) spaced from each other, and
the straight clamping lines (40) of all clamping locations (4) lie in one clamping plane (201), and
a transport device (7) for transporting the clamping device (3), by means of which a yarn sample (9) which is to be tested and is clamped at any of the clamping locations (4) is transportable to a predetermined testing position,
**characterized in that**
the yarn inlet eyelet (41) and the yarn clamps (42, 43) of each clamping location (4) are formed in such a way that the yarn sample (9) is insertable into the yarn inlet eyelet (41) and the yarn clamps (42, 43) in an insertion direction (202) which extends substantially perpendicularly to the straight clamping line (40) and perpendicularly to the clamping plane (201),
each yarn clamp (42, 43) contains a pair of clamping plates (51, 52) with clamping surfaces (511, 521) which are substantially parallel with respect to each other and interact for clamping the yarn sample (9), which clamping surfaces (511, 521) lie substantially perpendicularly to the clamping plane (201) and parallel to the straight clamping line (40), and
the yarn-changing device (2) contains an opening device (6) for the active opening of at least one of the two yarn clamps (42, 43).

2. A semiautomatic yarn-changing device (2) according to claim 1, wherein the clamping plates (51, 52) form an insertion slot (58) which is open against the insertion direction (202) and through which the yarn sample (9) are guidable between the clamping surfaces (511, 521).

3. A semiautomatic yarn-changing device (2) according to one of the preceding claims, wherein the clamping locations (4) are arranged on a flat carrier plate (30) which lies parallel to the clamping plane (201).

4. A semiautomatic yarn-changing device (2) according to claim 3, wherein the carrier plate (30) carries the yarn clamps (42, 43) and comprises a flange (33) which protrudes out of the plane of the carrier plate (30) and carries the yarn inlet eyelets (41).

5. A semiautomatic yarn-changing device (2) according to one of the preceding claims, wherein the yarn inlet eyelet (41) and the two yarn clamps (42, 43) are in alignment with each other in each clamping location (4).

6. A semiautomatic yarn-changing device (2) according to one of the preceding claims, wherein the transport device (7) is formed in such a way that it produces a linear displacement of the clamping device (4).

7. A semiautomatic yarn-changing device (2) according to one of the preceding claims, wherein the opening device (6) is formed for the active, independent opening of the two yarn clamps (42, 43) of the clamping location (4) which is disposed in the testing position.

8. A semiautomatic yarn-changing device (2) according to one of the preceding claims, wherein each yarn clamp (42, 43, 5) contains a pressure mechanism (53 to 57) for pressurising the clamping plates (51, 52) with a pressure force which presses the clamping surfaces (511, 521) against each other, which pressure mechanism (53-57) has
two arms (53, 54), of which each carries one of the two clamping plates (51, 52), a hinge (55) which rotatably connects the two arms via a hinge axis (56), and means (57) for exerting a torque on at least one of the two arms (53, 54).

9. A yarn-testing device (1) for the sequential testing of a plurality of yarn samples (9), comprising
a yarn path (12),
at least one sensor (19) arranged along the yarn path (12) for testing a yarn sample (9),
a semiautomatic yarn-changing device (2) for providing a plurality of yarn samples (9) for testing in the yarn-testing device (1) and
an automatic insertion device (13) for removing one of the yarn samples (9) provided in the semiautomatic yarn-changing device (2) and for its insertion into the yarn path (12),
**characterized in that**
the semiautomatic yarn-changing device (2) is a semiautomatic yarn-changing device according to one of the preceding claims.

10. A yarn-testing device (1) according to claim 9, wherein the clamping plane (201) is inclined in such a way that the yarn clamps (42, 43) of each clamping location (4) are situated at a higher altitude than the yarn inlet eyelet (41) of the same clamping location (4), and whose angle of inclination relative to the horizontal is between 0° and 45°, and preferably is 25°.

11. A yarn-testing device (1) according to claim 10, wherein the clamping locations (4) are arranged horizontally adjacent to each other and all yarn inlet eyelets (41) are disposed at the same height.

12. A yarn-testing device (1) according to one of the claims 9 to 11, wherein the semiautomatic yarn-changing device (2) is attached to a ceiling of the yarn-testing device (1) and is offset to the rear relative to a front (11) of the yarn-testing device (1) by at least one-third of the depth of the yarn-testing device (1).

13. A yarn-testing device (1) according to one of the claims 9 to 12, wherein the transport device (7) of the semiautomatic yarn-changing device (2) is formed in such a way that it produces a linear displacement of the clamping device (4) and the direction (76) of the linear displacement lies perpendicularly to the yarn path (12).

14. A yarn-testing device (1) according to claim 13, wherein the direction (76) of the linear displacement lies parallel to a front plane (111) defined by a front (11) of the yarn-testing device (1).

15. A yarn-testing device (1) according to one of the claims 9 to 14, wherein the automatic insertion device (13) comprises a gripper (14) for gripping the yarn sample (9) provided in the semiautomatic yarn-changing device (2), and the gripper (14) is displaceable on the one hand in a direction (101) which lies parallel to the yarn path (12) and is rotatable on the other hand about a rotational axis (102) which lies perpendicularly to the yarn path (12) and parallel to a front plane (111) which is defined by a front (11) of the yarn-testing device (1).

## Revendications

1. Dispositif semi-automatique de changement de fil (2) pour la préparation de plusieurs échantillons de fil (9) en vue du contrôle dans un appareil de contrôle de fils (1), comprenant
une installation de serrage (3) avec plusieurs points de serrage (4) disposés les uns à côté des autres dans des positions fixes à distance les uns des autres, pour le serrage des échantillons de fil (9),
chaque point de serrage (4) comprenant un oeillet d'entrée de fil (41) et deux pinces à fil (42, 43) disposées dans l'alignement l'une de l'autre en aval de l'oeillet d'entrée de fil (41),
l'oeillet d'entrée de fil (41) et les deux pinces à fil (42, 43) de chaque point de serrage (4) se trouvant à distance l'un des autres sensiblement sur une droite de serrage (40) et
le droite de serrage (40) de tous les points de serrage (4) se trouvant dans un plan de serrage (201), et
une installation de transport (7) pour le transport de l'installation de serrage (3), de sorte qu'un échantillon de fil (9) à contrôler serré sur l'un quelconque des points de serrage (4) peut être transporté dans une position de contrôle prédéterminée,
**caractérisé en ce que**
l'oeillet d'entrée de fil (41) et les pinces à fil (42, 43) de chaque point de serrage (4) sont conçus de telle sorte que l'échantillon de fil (9) peut être introduit dans l'oeillet d'entrée de fil (41) et les pinces à fil (42, 43) dans une direction d'introduction (202) sensiblement perpendiculaire à la droite de serrage (40) et perpendiculaire au plan de serrage (201),
chaque pince à fil (42, 43) comporte une paire de plaquettes de serrage (51, 52) avec des surfaces de serrage (511, 521) sensiblement parallèles coopérant pour serrer l'échantillon de fil (9), lesquelles surfaces de serrage (511, 521) sont sensiblement perpendiculaires au plan de serrage (201) et parallèles à la droite de serrage (40), et le dispositif de changement de fil (2) comprend une installation d'ouverture (6) pour l'ouverture active d'au moins une des deux pinces à fil (42, 43).

2. Dispositif semi-automatique de changement de fil (2) selon la revendication 1, dans lequel les plaquettes de serrage (51, 52) forment une fente d'introduction (58) ouverte en sens inverse de la direction d'introduction (202), à travers laquelle l'échantillon de fil (9) peut être guidé entre les surfaces de serrage (511, 521).

3. Dispositif semi-automatique de changement de fil (2) selon l'une des revendications précédentes, dans lequel les points de serrage (4) sont disposés sur une plaque de support plane (30) parallèle au plan de serrage (201).

4. Dispositif semi-automatique de changement de fil (2) selon la revendication 3, dans lequel la plaque de support (30) porte les pinces à fil (42, 43) et présente une bride (33) qui dépasse du plan de la plaque de support (30) et qui porte les oeillets d'entrée de fil (41).

5. Dispositif semi-automatique de changement de fil (2) selon l'une des revendications précédentes, dans lequel l'oeillet d'entrée de fil (41) et les deux pinces à fil (42, 43) sont alignés les uns sur les autres à chaque point de serrage (4).

6. Dispositif semi-automatique de changement de fil (2) selon l'une des revendications précédentes, dans lequel l'installation de transport (7) est conformée de manière à produire une translation linéaire de l'installation de serrage (4).

7. Dispositif semi-automatique de changement de fil (2) selon l'une des revendications précédentes, dans lequel l'installation d'ouverture (6) est conçue pour l'ouverture active et indépendante des deux pinces à fil (42, 43) du point de serrage (4) qui se trouve dans la position de contrôle.

8. Dispositif semi-automatique de changement de fil (2) selon l'une des revendications précédentes, dans lequel chaque pince à fil (42, 43, 5) comprend un mécanisme presseur (53-57) pour exercer sur les plaquettes de serrage (51, 52) une force de pression écartant les surfaces de serrage (511, 521) l'une de l'autre, lequel mécanisme presseur (53-57) comporte
deux bras (53, 54) dont chacun porte l'une des deux plaquettes de serrage (51, 52),
une charnière (55) qui relie les deux bras de façon pivotante sur un axe de charnière (56) et
des moyens (57) pour exercer un couple de rotation sur au moins un des deux bras (53, 54).

9. Appareil de contrôle de fils (1) pour le contrôle séquentiel d'un grand nombre d'échantillons de fil (9), avec
un trajet de fil (12),
au moins un capteur (19) disposé le long du trajet de fil (12) pour contrôler un échantillon de fil (9),
un dispositif de changement de fil semi-automatique (2) pour fournir le grand nombre d'échantillons de fil (9) pour le contrôle dans l'appareil de contrôle de fils (1) et
un dispositif d'insertion automatique (13) pour prélever l'un des échantillons de fil (9) présentés dans le dispositif de changement de fil semi-automatique (2) et l'insérer dans le trajet de fil (12),
**caractérisé en ce que**
le dispositif de changement de fil semi-automatique (2) est un dispositif de changement de fil semi-automatique selon l'une des revendications précédentes.

10. Appareil de contrôle de fils (1) selon la revendication 9, dans lequel le plan de serrage (201) est incliné de telle manière que les pinces à fil (42, 43) de chaque point de serrage (4) se trouvent plus haut que l'oeillet d'entrée de fil (41) du même point de serrage (4) et leur angle d'inclinaison par rapport à l'horizontale est compris entre 0° et 45° et de préférence de 25°.

11. Appareil de contrôle de fils (1) selon la revendication 10, dans lequel les points de serrage (4) sont disposés horizontalement les uns à côté des autres et tous les oeillets d'entrée de fil (41) se trouvent à la même hauteur.

12. Appareil de contrôle de fils (1) selon l'une des revendications 9 à 11, dans lequel le dispositif de changement de fil semi-automatique (2) est disposé sur une face supérieure de l'appareil de contrôle de fils (1) et décalé vers l'arrière par rapport à une façade (11) de l'appareil de contrôle de fils (1) d'au moins un tiers de la profondeur de l'appareil de contrôle de fils (1).

13. Appareil de contrôle de fils (1) selon l'une des revendications 9 à 12, dans lequel l'installation de transport (7) du dispositif de changement de fil semi-automatique (2) est conformé de manière à produire une translation linéaire de l'installation de serrage (4) et la direction (76) de la translation linéaire est perpendiculaire au trajet de fil (12).

14. Appareil de contrôle de fils (1) selon la revendication 13, dans lequel la direction (76) de la translation linéaire est parallèle à un plan de façade (111) défini par une façade (11) de l'appareil de contrôle de fils (1).

15. Appareil de contrôle de fils (1) selon l'une des revendications 9 à 14, dans lequel le dispositif d'insertion automatique (13) comprend une pince (14) pour saisir l'échantillon de fil (9) présenté dans le dispositif de changement de fil semi-automatique (2) et la pince (14) est capable, d'une part, de translation dans une direction (101) parallèle au trajet de fil (12) et, d'autre part, de rotation autour d'un axe de rotation (102) qui est perpendiculaire au trajet de fil (12) et parallèle à un plan de façade (111) défini par une façade (11) de l'appareil de contrôle de fils (1).
